(19) Europäisches Patentamt
European Patent Office
Office européen des brevets

(11) Publication number: 0 071 358
B1

(12) # EUROPEAN PATENT SPECIFICATION

(45) Date of publication of patent specification: **09.04.86**

(51) Int. Cl.$^4$: **C 07 D 491/052** //
(C07D491/052, 311:00,
249:00)

(21) Application number: **82303660.3**

(22) Date of filing: **13.07.82**

(54) **Benzopyrano(2,3-d)-v-triazole intermediates.**

(30) Priority: **16.07.81 GB 8122022**

(43) Date of publication of application:
**09.02.83 Bulletin 83/06**

(45) Publication of the grant of the patent:
**09.04.86 Bulletin 86/15**

(84) Designated Contracting States:
**BE CH DE FR GB IT LI NL SE**

(56) References cited:
**EP-A-0 054 398**

(73) Proprietor: **BEECHAM GROUP PLC**
**Beecham House Great West Road**
**Brentford Middlesex TW8 9BD (GB)**

(72) Inventor: **Buckle, Derek Richard**
**18 Hillfield Road**
**Redhill Surrey (GB)**

(74) Representative: **Russell, Brian John et al**
**European Patent Attorney**
**Beecham Pharmaceuticals**
**Great Burgh Yew Tree Bottom Road**
**Epsom Surrey, KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 071 358 B1

**Description**

This invention relates to benzopyrano [2,3-d]-v-triazole intermediates, to a process for their preparation, and for their use in the preparation of compounds having pharmacological activity.

Published European Patent Application No. 0,034,004 discloses that compounds of the formula (A), and pharmaceutically acceptable salts thereof:

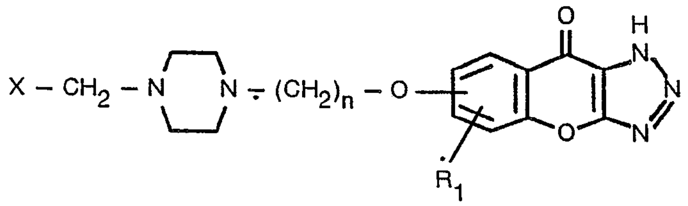

(A)

wherein X is phenyl, optionally substituted by one halogen, $C_{1-4}$ alkyl or $C_{1-4}$ alkoxy; or pyridyl; $R_1$ is hydrogen or $C_{1-6}$ alkyl; and n is 1 to 6; may be used in the prophylaxis or treatment of diseases the symptoms of which are controlled by the mediators of the allergic response, for example asthma, hay-fever, rhinitis and allergic eczema. This European Patent Application also discloses a process for the preparation of the compounds of formula (A) from compounds of formula (B):

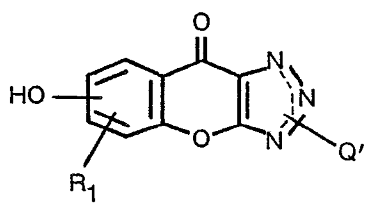

(B)

wherein $R_1$ is as defined in formula (A), and Q' is a N-protecting group, such as labile benzyl groups, for example $C_{1-6}$ alkoxy substituted benzyl groups, particularly 4-methoxy benzyl. With Q' as 4-methoxy benzyl, as exemplified in the European Patent Application, the preparation of a compound of formula (B) yields a mixture of isomers, which mixture may be used in the form of this mixture, or may first be separated into the individual isomers.

Intermediates for the compounds of formula (A) have now been discovered which may be prepared directly in the form of one isomer, with corresponding advantages in ease of purification and characterisation.

Accordingly the present invention provides a compound of formula (I):

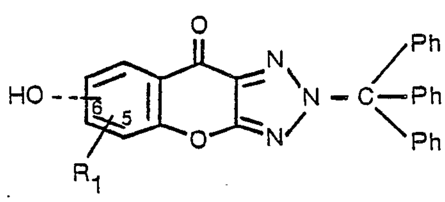

(I)

wherein $R_1$ is as defined in formula (A), and each phenyl group is optionally substituted by one or two inert substituents selected from $C_{1-3}$ alkyl, $C_{1-3}$ alkoxy and halogen.

In formula (I) any substituent present in a phenyl is most suitably para- or meta-. $C_{1-3}$ alkyl substituents are suitably methyl; $C_{1-3}$ alkoxy substituents are suitably methoxy; halogen substituents are suitably bromine or chlorine. In general, when substituents are present in phenyl groups, then not more than one substituent on each phenyl is present. Preferably the trityl group in formula (I) is not substituted.

Suitable examples of $R_1$ include hydrogen, methyl, ethyl, and *n*- and *iso*-propyl. When other than hydrogen, $R_1$ is suitably in the 5-position. Preferably $R_1$ is hydrogen or 5-methyl.

Suitably the HO— shown in formula (I) substitutes the ring in the 6-position.

Compounds of formula (I) may be prepared by reacting a compound of formula (II):

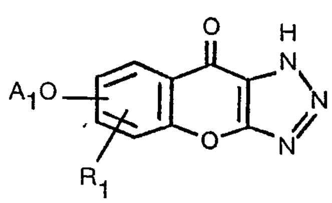

(II)

wherein $R_1$ is defined in formula (A); and $A_1$ is hydrogen, or an oxygen protecting group, with a triphenylmethyl halide wherein the phenyls may be substituted by inert substituents as hereinbefore described.

The reaction is suitably carried out in the presence of a moderate base in a polar solvent. Examples of suitable bases include alkali metal salts such as the carbonates, for instance potassium carbonate. Examples of suitable solvents include N,N-dimethylformamide.

The reaction is usually carried out between 20—80°C and for times between 2 and 24 hours, the reaction being followed by tlc.

If an oxygen protected derivative of the compound of formula (II) is used, then suitable examples of such protecting groups include sulphonyl groups such as mesyl and tosyl; and acyl groups, such as acetyl. These groups, when present, may be removed after the reaction in conventional manner, for example in alkaline conditions.

Compounds of the formula (II) may be prepared by the de-alkylation of a compound of formula (III):

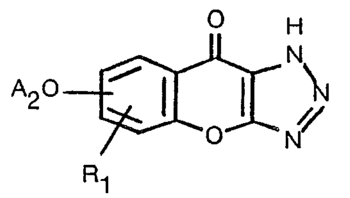

(III)

wherein $R_1$ is as defined in formula (A) and $A_2$ is $C_{1-4}$ alkyl, and thereafter if required, O-protection. This de-alkylation may suitably be effected with a base such as sodium thioethoxide in a polar solvent such as dimethylformamide, at moderately elevated temperatures, following the procedure of Feutrill et al, Tet Letters, 1970, 1327. The optional O-protection can be carried out in any convenient manner. For example we have found that sulphonyl and acyl functions selectively react at the hydroxyl functions, and thus are very suitable for use.

Compounds of formula (III), and their preparation, are disclosed in published European Patent Application No: 79301318.6, Publication No: 0 007 727. (Compounds of formula (II), and their preparation, are also disclosed in published European Patent Application No. 0,032,821.

The compounds of formula (I) are useful as intermediates in the preparation of compounds of the formula (A). The synthetic sequence resulting in the compounds of formula (A) follows in outline that described in the published European Patent Application No. 0,032,821 referred to above, but has advantages thereover.

Firstly, the compound of formula (I) is coupled to a compound of formula (IV):

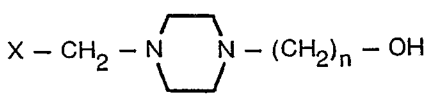

(IV)

wherein X and n are as in formula (A), to yield a compound of formula (V):

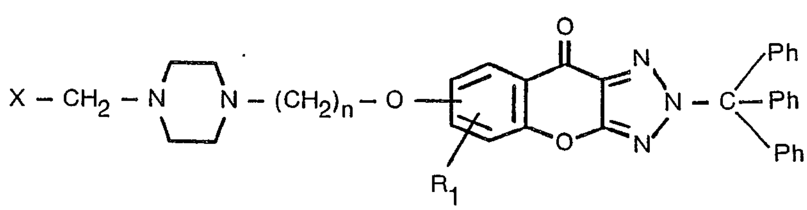

(V)

wherein, as before, the trityl phenyls may be substituted. Compounds of formula (V) are described and claimed in our co-pending divisional European Patent Application No. 84116176.3, (EP—A—149223).

This reaction is generally carried out in the presence of a compound of formula (VI):

$$R_3O_2C—N=N—CO_2R_4 \quad (VI)$$

wherein $R_3$ and $R_4$ are independently $C_{1-6}$ alkyl, aryl or aryl-$C_{1-6}$ alkyl, generally both ethyl, and a compound of formula (VII):

$$PR_5R_6R_7 \quad (VII)$$

wherein $R_5$, $R_6$ and $R_7$ are independently $C_{1-6}$ alkyl, $C_{1-6}$ alkoxy, aryl, aryloxy, aryl-$C_{1-6}$ alkyl or aryl-$C_{1-6}$ alkoxy, generally all phenyl.

The reaction is generally carried out at a non-extreme temperature, such as −20 to 100°C, in an inert aprotic organic solvent such as tetrahydrofuran, dioxan, ethyl acetate or benzene.

Compounds of formula (IV) are either known compounds, or may be prepared in analogous manner to known compounds.

Compounds of formula (V) are de-protected to yield the desired end products of formula (A).

This de-protection may be achieved extremely readily, an important advantage of this invention, with acid, for example with trifluoroacetic acid. The de-protection can also be achieved using hydrochloric acid in acetic acid at slightly raised temperatures (eg 55°C).

Suitable and preferred examples of the compounds of formula (A) (and of the variables therein) for preparation in the manner of this invention are as described in published European Patent Application No. 0,034,004. Thus for the sake of completeness, in compounds of formula (A):

When X is an optionally substituted phenyl group, then it may be represented:

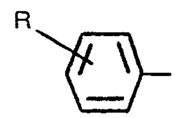

wherein R is hydrogen, halogen, $C_{1-4}$ alkyl or $C_{1-4}$ alkoxy. Suitable examples of R include hydrogen, fluorine, chlorine, bromine, methyl, ethyl, methoxy and ethoxy. More suitably R is hydrogen or halogen, preferably chlorine. Preferably R when halogen is in the *o*- or *p*-position and most preferably is *p*-chloro.

Alternatively X may be pyridyl. In such cases X is suitably 2- or 4-pyridyl, preferably 2-pyridyl.

Suitable examples of $R_1$ include hydrogen, methyl, ethyl and *n*- and *iso*-propyl. When other than hydrogen, $R_1$ is suitably in the 5-position (that is, substituting the carbon atom adjacent the oxygen atom joined bridgehead carbon atom). Preferably $R_1$ is hydrogen or 5-methyl.

n is suitably 2, 3 or 4, preferably 3.

The side chain oxygen atom may join the benzopyrano[2,3-d]-v-triazole nucleus at any non-bridgehead carbon in the benzo moiety. Suitably however it will be joined at the 6-position (that is, substituting the carbon atom *meta*- to the oxygen atom joined bridgehead carbon atom).

Within formula (A) there is a group of compounds of formula (A)′:

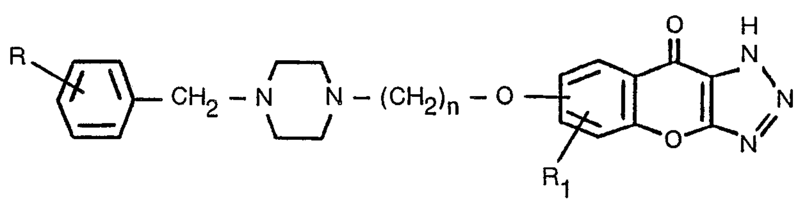

(A)′

wherein R is hydrogen, halogen, $C_{1-4}$ alkyl or $C_{1-4}$ alkoxy; $R_1$ is hydrogen or $C_{1-6}$ alkyl; and n is 1 to 6.

Suitable and preferred examples of the variable groups therein are as described in relation to formula (*A*).

Within formula (A) there is also a group of compounds of formula (A)″:

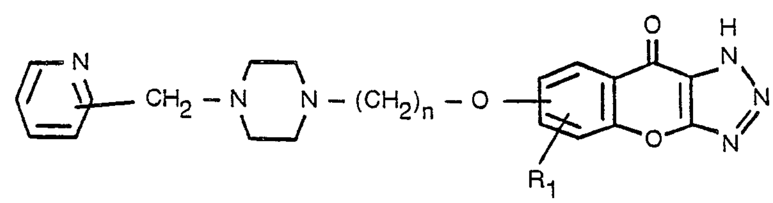

(A)″

wherein $R_1$ is hydrogen or $C_{1-6}$ alkyl; and n is 1 to 6.

Suitable and preferred examples of the variable groups therein are as described in relation to formula (A).

From the aforesaid it will be appreciated that one preferred sub-group of compounds of the formula (A) is of formula (A)‴:

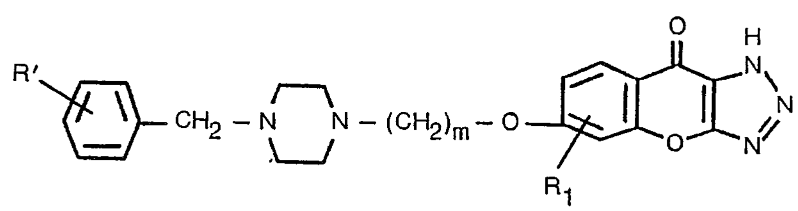

(A)‴

wherein R′ is hydrogen or halogen, $R_1$ is hydrogen or $C_{1-6}$ alkyl, and m is 2, 3 or 4.

Suitable and preferred values for R′, $R_1$ and m are as hereinbefore described for R, $R_1$ and n respectively.

Thus R′ when halogen is preferably in the *o*- or *p* position, most preferably *p*-chloro.

Similarly $R_1$ when alkyl is preferably in the 5-position, preferably hydrogen or 5-methyl.

The triazole moiety of the compounds of formula (A) has an acidic hydrogen, and accordingly may form salts. Examples of pharmaceutically acceptable salts falling within the scope of this invention include the aluminium, alkali metal and alkaline earth metal salts such as the sodium, potassium and magnesium salts; and salts with ammonia, organic bases and amino compounds. The compounds of the formula (A) may also form salts of the amino moieties, such as salts with hydrochloric and sulphuric acid.

Particularly preferred compounds of formula (A) for preparation are:

8-{3-[4-(4-chlorobenzyl)-1-piperazinyl]propyloxy}-9-oxo-1H,9H-benzopyrano[2,3-d]-v-triazole;

6-{3-[4-(4-chlorobenzyl)-1-piperazinyl]propyloxy}-9-oxo-5-n-propyl-1H,9H-benzopyrano[2,3-d]-v-triazole;

6-{3-[4-(4-chlorobenzyl)-1-piperazinyl]propyloxy}-5-methyl-9-oxo-1H,9H-benzopyrano[2,3-d]-v-triazole; and

6-{3-[4-(4-chlorobenzyl)-1-piperazinyl]propyloxy}-9-oxo-1H,9H-benzopyrano[2,3-d]-v-triazole, and pharmaceutically acceptable salts thereof.

The following Examples illustrate the invention.

## Example 1

(a) Ethyl 1-benzyl-5-(3-methoxy-2-methylphenoxy)-v-triazole-4-carboxylate

To a stirred solution of 3-methoxy-2-methylphenol (13.8 g, 0.1 mol) in dry DMF (100 mL) was added a 50% dispersion of NaH in mineral oil (4.8 g, 0.1 mol) and the solution was stirred for 1 hour at room temperature to ensure formation of the sodium salt. To this solution was added ethyl 1-benzyl-5-chloro-v-triazole-4-carboxylate (26.6 g, 0.1 mol) in a single portion and the mixture stirred at 70—80°C for 24 hours. After cooling, the solvent was removed *in vacuo* and the residue partitioned between EtOAc and water. The organic phase was washed with dilute aqueous NaOH, brine, and dried ($MgSO_4$). Evaporation of the solvent gave an oil which was chromatographed on $SiO_2$ eluting with chloroform to give 30.8 g (84%) of the title compound as an oil which was not further purified, $\nu_{max}$ (film) 1722 $cm^{-1}$ (C = O); $^1H$ nmr ($CDCl_3$) δ 1.03 (3H, t, J = 7 Hz, ester $CH_3$), 2.18 (3H, s, aromatic $CH_3$), 3.85 (3H, s, $OCH_3$), 4.11 (2H, q, J = 7 Hz, ester $CH_2$), 5.37 (2H, s, benzyl $CH_2$), 5.89 (1H, d, J = 8.5 Hz, C—4H), 6.59 (1H, d, J = 8.5 Hz, C—6H), 6.88 (1H, t, J = 8.5 Hz, C—5H), 7.29 (5H, s, Ph).

(b) Ethyl 5-(3-methoxy-2-methylphenoxy)-1H-v-triazole-4-carboxylate

A solution of the above ester (30 g, 0.082 mol) in EtOH (400 mL) was hydrogenated at 96°C and 1,000 psi over 10% palladinised charcoal (0.3 g) until the reaction was complete (2—3 hours). The catalyst was removed from the cooled solution by filtration and the filtrate evaporated to an oil which crystallised after chromatography on $SiO_2$ eluting with chloroform. Recrystallisation from toluene-petroleum ether [b.p. 60—80°C] gave 20.6 g (91%) of the title ester of m.p. 107—108°C, $\nu_{max}$ (mull) 3150 (broad, N—H), 1705 $cm^{-1}$ (C = O); $^1H$ nmr ($CDCl_3$) δ 1.26 (3H, t, J = 7 Hz, ester $CH_3$), 2.12 (3H, s, aromatic $CH_3$), 3.81 (3H, s, $OCH_3$), 4.32 (2H, q, J = 7 Hz, ester $CH_2$), 6.63 (2H, distorted d, C—4 and 6H), 7.09 (1H, m, C—5H), 10.3 (1H, broad, exchangeable, N—H).

(Found: C, 56.28; H, 5.13; N, 5.18; $C_{13}H_{15}N_3O_4$ requires C, 56.31; H, 5.45; N, 5.16%).

(c) 5-(3-Methoxy-2-methylphenoxy)-1H-v-triazole-4-carboxylic acid

Hydrolysis of the above ester (19.4 g, 0.07 mol) with 1.25M aqueous NaOH (220 mL) at 70—80°C for three hours gave on cooling and acidification 12.9 g (74%) of the title acid. Recrystallisation from EtOAc-petroleum ether [b.p. 60—80°C] gave material of m.p. 129°C (dec) after initial loss of water of crystallisation at *ca* 90°C, $\nu_{max}$ (mull) 1685 $cm^{-1}$ (C = O); $^1H$ nmr ($Me_2SO$-$d_6$) δ 2.10 (3H, s, aromatic $CH_3$), 3.82 (3H, s, $OCH_3$), 6.52 (1H, d, J = 8.5 Hz), 6.76 (1H, d, J = 8.5 Hz), 7.12 (1H, t, J = 8.5 Hz, C—5H).

Found: C, 50.64; H, 4.65; N, 15.59; $C_{11}H_{11}N_3O_4 \cdot 0.75H_2O$ requires C, 50.28; H, 4.80; N, 15.99%.

(d) 6-Methoxy-5-methyl-9-oxo-1H,9H-benzopyrano[2,3-d]-v-triazole

*(Method A)*

Phosphoric oxide (240 g) was added to vigorously stirred 98% methanesulphonic acid (600 mL) at 80°C (oil bath temperature) and the mixture stirred until homogenous (1 hour). Powdered 5-(3-methoxy-2-methylphenoxy)-1H-v-triazole-4-carboxylic acid (32.30 g, 0.13 mol) was added and the mixture stirred for 90 minutes at 80°C when the mixture was cooled and poured into cold water. After 16 hours the precipitated product was filtered off, washed well with water and recrystallised from EtOH to give the title triazole (19.80 g, 66%) of mp 268—270°C (dec), $\nu_{max}$ (mull) 3300 (broad, N—H), 1638 $cm^{-1}$ (C = O); $^1H$ nmr ($Me_2SO$-$d_6$) δ 2.26 (3H, s, aromatic $CH_3$), 3.97 (3H, s, $OCH_3$), 7.12 (1H, d, J = 9 Hz, C—7H), 8.02 (1H, d, J = 9 Hz, C—8H), 16.5 (1H, broad, exchangeable N—H).

Found: C, 57.30; H, 3.99; N, 18.47; $C_{11}H_9N_3O_2$ requires C, 57.14; H, 3.92; N, 18.18%.

Evaporation of the ethanolic mother liquors afforded 3.4 g of solid shown by TLC ($CHCl_3$, 10% MeOH) to be a mixture of product and a faster ($R_f$ *ca* 0.8) material. Chromatography on $SiO_2$ eluting with

chloroform gave 1.32 g of a white crystalline solid of mp 203°C (from EtOH) shown to be 3-diazo-7-methoxy-8-methylcoumarin-4-oxide: $\nu_{max}$ (mull) 2180 ($N_2^+$) 1720 $cm^{-1}$ (C = O); $^1H$ nmr ($CDCl_3$) δ 2.28 (3H, s, aromatic $CH_3$), 3.98 (3H, s, $OCH_3$), 6.83 (1H, d, J = 9 Hz, C—6H), 7.90 (1H, d, J = 9 Hz, C—5H).

Found: C, 57.23; H, 3.33; N, 12.27; $C_{11}H_8N_2O_4$ requires: C, 56.90; H, 3.47; N, 12.07%.

Further elution with 30% MeOH in chloroform resulted in an additional 1.92 g (6%) of title compound.

*(Method B)*

Powdered 5-(3-methoxy-2-methylphenoxy)-1H-v-triazole-4-carboxylic acid (0.55 g) was added to 85% polyphosphoric acid (5.5 g) and the mixture was vigorously stirred at 100°C for 3.75 hours when hplc monitoring indicated complete reaction. The cooled mixture was diluted with ice-water and allowed to stand overnight. Filtration of the precipitated solid afforded 0.36 g (73%) of material identical with that prepared above and pure by tlc and nmr.

(e) 6-hydroxy-5-methyl-9-oxo-1H,9H-benzopyrano[2,3-d]-v-triazole

To a solution of ethanethiol (2.55 g, 3.01 ml, 0.04 mole) in dry N,N-dimethylformamide (50 ml) was added a 50% dispersion of sodium hydride in mineral oil (1.92 g, 0.04 mole) and the mixture was stirred for 15 minutes to complete the formation of the sodium salt. Finely powdered 6-methoxy-5-methyl-9-oxo-1H,9H-benzopyrano[2,3-d]-v-triazole (1.27 g, 5.5 mmole) was then added and the temperature brought to 115°C (oil bath temperature) for 90 minutes. After cooling the mixture was poured into water, extracted once with ether and then acidified with dilute hydrochloric acid. Crystals slowly separated on cooling which were filtered off, washed with water and dried. Recrystallisation from ethyl acetate-ethanol (with charcoal treatment) gave 1.188 g (99%) of title compound of mp 292—293°C (dec), $\nu_{max}$ (mull) 3170, 2600 (broad), 1645, 1610, 1560, 1545 $cm^{-1}$, δ (DMSO-$d_6$) 2.29 (3H, s); 6.97 (1H, d, J 9 Hz); 7.90 (1H, d, J 9 Hz); 11.00 (1H, exchangeable).

(Found: C, 55.31; H, 3.03; N, 18.78; $C_{10}H_7N_3O_3$ requires: C, 55.30; H, 3.25; N, 19.35%). $M^+$ ($C_{10}H_7N_3O_3$) 217.0489.

(f) 6-Hydroxy-5-methyl-9-oxo-2-triphenylmethyl-9H-benzopyrano[2,3-d]-v-triazole

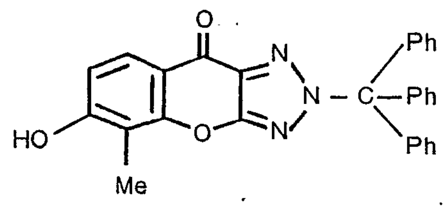

A mixture of 6-hydroxy-5-methyl-9-oxo-1H,9H-benzopyrano[2,3-d]-v-triazole (0.868 g, 4 mmole), anhydrous potassium carbonate (0.276 g, 2 mmole) and dry N,N-dimethylformamide (25 ml) was stirred at 60°C for 10 minutes and triphenylmethyl chloride (1.114 g, 4 mmole) was added. After a further 24 hours at this temperature the solvent was removed in vacuo and water was added. The insoluble buff solid was filtered off, washed with water and dried in vacuo over $P_2O_5$. Chromatography on silica gel eluting with chloroform then gave 1.09 g (59%) of material of mp (methanol-chloroform) 282°C (dec), $\nu_{max}$ (mull) 3260, 1650, 1598, 1590, 1545 $cm^{-1}$; δ (DMSO-$d_6$) 2.25 (3H, s); 6.97 (1H, d, J 9 Hz); 7.22 (15H, m); 7.90 (1H, d, J 9 Hz); 11.00 (1H, sharp exchangeable s).

(Found: C, 76.19; H, 4.41; N, 8.89; $C_{29}H_{21}N_3O_3$ requires: C, 75.80; H, 4.61; N, 9.15%).

(g) 6-{3-[4-(4-Chlorobenzyl)-1-piperazinyl]-propyloxy}-5-methyl-9-oxo-2-triphenylmethyl-9H-benzopyrano[2,3-d]-v-triazole

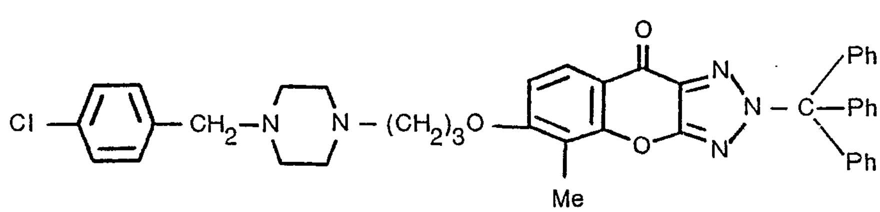

A mixture of 6-hydroxy-5-methyl-9-oxo-2-triphenylmethyl-9H-benzopyrano[2,3-d]-v-triazole (230 mg, 0.5 mmole), 1-(4-chlorobenzyl)-4-(3-hydroxypropyl)piperazine (135 mg, 0.5 mmole) and triphenyl phosphine (200 mg) in dry tetrahydrofuran (20 ml) was stirred at room temperature and a solution of diethyl azodiicarboxylate (180 mg) in dry tetrahydrofuran (2 ml) added over two minutes. After 1 hour at room temperature a further addition of triphenyl phosphine (200 mg) and diethyl azodiicarboxylate (180 mg) was made and the solvent evaporated after an additional hour. The residual gum was chromatographed on silica eluting with chloroform to afford 120 mg (34%) of title compound of $R_f$ 0.2 as a

foam, δ ($CDCl_3$) 2.05 (2H, quintet, J 6.2 Hz), 2.33 (3H, s), 2.50 (8H, broad singlet), 2.58 (2H, t, J 6.2 Hz), 3.49 (2H, s), 4.18 (2H, t, J 6.2 Hz), 6.69 (1H, d, J 9.5 Hz), 7.26 (19H, complex m), 8.22 (1H, d, J 9.5 Hz).

(h) 6-{3-[4-(4-Chlorobenzyl)-1-piperazinyl]propyloxy}-5-methyl-9-oxo-1H,9H-benzopyrano[2,3-d]-v-triazole

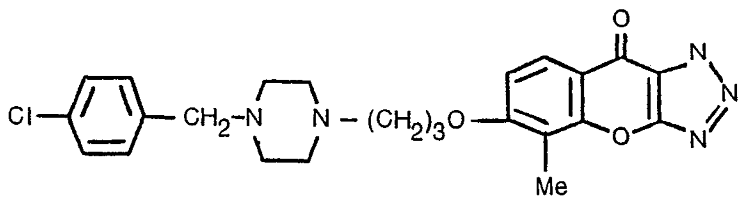

Concentrated hydrochloric acid (1 ml) was added to a stirred solution of 6-{3-[4-(4-chlorobenzyl)-1-piperazinyl]-propyloxy}-5-methyl-9-oxo-2-triphenylmethyl-9H-benzopyrano[2,3-d]-v-triazole (100 mg) in glacial acetic acid (5 ml) and the solution stirred at 50°C for 1 hour. During this time some of the title compound separated as its dihydrochloride which was filtered off after cooling. Evaporation of the solvent and trituration with methanol gave a further crop of material so that the isolated yield was 57 mg (75%) of compound of mp 252—254°C (dec), $v_{max}$ (KBr) 3100—2300 (broad), 1673, 1610, 1554, 1455, 1417, 1280, 1102 $cm^{-1}$.

(Found: C, 53.07; H, 5.17; N, 12.76; $C_{24}H_{28}N_5Cl_3O_3$ requires: C, 53.29; H, 5.22; N, 13.08%.)

Further product was evident in the methanolic mother liquors (by hplc analysis) but was not isolated. The material was coincident with authentic material on hplc and could be converted to the known free compound by basification to pH 6.

Example 2

(a) Ethyl 1-benzyl-5-(3-methoxyphenoxy)-v-triazole-4-carboxylate

A 50% dispersion of sodium hydride in mineral oil (6.40 g, 0.13 mole) was added to a stirred solution of 3-methoxyphenol (16.40 g, 0.13 mole), in dry DMF (500 ml) and to the resulting sodium salt was added ethyl 1-benzyl-5-chloro-v-triazole-4-carboxylate (35.0 g, 0.13 mole). The reaction mixture was heated with stirring at 70°C for 21 hours and cooled.

Removal of the DMF *in vacuo* gave a brown oil which was taken up in ether, washed with 1M sodium hydroxide solution, water and brine and dried ($MgSO_4$). Evaporation gave a red oil which crystallised on trituration with ether/petrol ether [40—60°C] (1:1).

Recrystallisation from ether/petrol ether [60—80°C] gave 33.75 g (53%) of product of mp 52—53°C. (Found: C, 64.51; H, 5.27; N, 12.05; $C_{19}H_{19}N_3O_4$ requires: C, 64.58; H, 5.42; N, 11.89%).

(b) Ethyl 5-(3-methoxyphenoxy)-1H-v-triazole-4-carboxylate

Hydrogenolysis of a solution of ethyl 1-benzyl-5-(3-methoxyphenoxy)-v-triazole-4-carboxylate (17.27 g) in ethanol (300 ml), over 10% palladium on charcoal at 100°C and 1000 psi resulted in clean removal of the N-benzyl group in 2.5 hours. On cooling and removal of the catalyst by filtration, evaporation of the solvent gave an orange oil which crystallised on trituration.

Recrystallisation from ethanol/water gave 9.16 g (71%) of product of mp 104—5°C. (Found: C, 54.92; H, 4.88; N, 15.71; $C_{12}H_{13}N_3O_4$ requires C, 54.75; H, 4.98; N, 15.96%).

(c) 5-(3-Methoxyphenoxy)-1H-v-triazole-4-carboxylic acid

Hydrolysis of ethyl 5-(3-methoxyphenoxy)-1H-v-triazole-4-carboxylate (9.10 g), with 5% aqueous sodium hydroxide (110 ml), at 80°C for 1 hour afforded the acid which was isolated after acidification of the cooled (0°C) solution. Recrystallisation from ethyl acetate/petrol ether [40—60°C] gave 6.20 g (76%) of material of mp 133—4°C (dec). (Found: C, 51.31; H, 3.60; N, 17.88. $C_{10}H_9N_3O_4$ requires: C, 51.07; H, 3.86; N, 17.86%).

(d) 6-Methoxy-9-oxo-1H,9H-benzopyrano[2,3-d]-v-triazole and
8-Methoxy-9-oxo-1H,9H-benzopyrano[2,3-d]-v-triazole

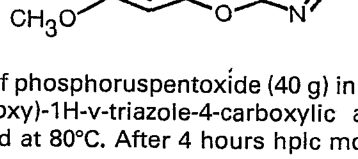

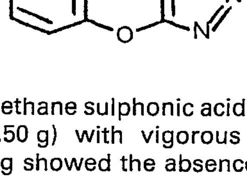

To a solution of phosphoruspentoxide (40 g) in 98% methane sulphonic acid (100 g) at 80°C was added 5-(3-methoxyphenoxy)-1H-v-triazole-4-carboxylic acid (5.50 g) with vigorous stirring and the brown solution maintained at 80°C. After 4 hours hplc monitoring showed the absence of starting material. The

solution was cooled, diluted with ice water and left to stand overnight. The pink precipitate was collected by filtration to yield 4.1 g, (75%) of the 6-methoxy and 8-methoxy mixed isomers in the ratio 2:1 respectively. The pure isomers could be separated by fractional crystallisation from ethanol.

Data for pure 6-methoxy isomer

mp 270—1°C (dec), $v_{max}$ (mull) 1585, 1615, 1640, 1655 $cm^{-1}$, δ $DMSOd_6$; 3.94 (3H, s), 7.09 (1H, dd, *J* 2 & 7 Hz), 7.27 (1H, d, *J* 2 Hz), 8.12 (1H, d, *J* 7 Hz). (Found: C, 54.97; H, 3.54; N, 19.51; $C_{10}H_7N_3O_3$ requires: C, 55.30; H, 3.25; N, 19.35%). $M^+ = 217.0494$ ($C_{10}H_7N_3O_3$).

Data for pure 8-methoxy isomer

mp 258—60°C, $v_{max}$ (mull) 1550, 1610, 1658, 1670, 3130 $cm^{-1}$; δ $DMSOd_6$; 3.92 (3H, s), 7.06 (1H, dd, *J* 8 Hz), 7.24 (1H, dd, *J* 8 Hz), 7.77 (1H, t, *J* 8 Hz). (Found: C, 55.31; H, 3.29; N, 19.64; $C_{10}H_7N_3O_3$ requires C, 55.30; H, 3.25; N, 19.35%). $M^+ = 217.0487$ ($C_{10}H_7N_3O_3$).

(e) 6-Hydroxy-9-oxo-1H,9H-benzopyrano[2,3-d]-v-triazole

A 50% dispersion of sodium hydride in mineral oil (4.30 g, 0.09 mole) was added to a stirred solution of ethanethiol (5.46 g, 0.09 mole) in dry DMF (90 ml) and to the resulting sodium salt was added 6-methoxy-9-oxo-1H,9H-benzopyrano[2,3-d]-v-triazole (2.25 g, 0.01 mole). The mixture was heated with stirring to 120°C for 1.5 h when TLC showed no starting material. The reaction was cooled and poured into ice-water. The product was isolated by acification of the solution, filtration and recrystallisation from aqueous DMF to give 1.80 g (85%) of material of mp 300°C (dec); $v_{max}$ (mull) 1550, 1580, 1650, 3215 $cm^{-1}$; δ (DMSO-$d_6$) 6.94 (2H, m), 8.08 (1H, d, J 9 Hz), 11.01 (1H, s, exchanges with $D_2O$). $M^+ = 203.0337$ ($C_9H_5N_3O_3$).

(f) 6-Hydroxy-9-oxo-2-triphenylmethyl-9H-benzopyrano[2,3-d]-v-triazole

Anhydrous potassium carbonate (0.89 g) was added to a solution of 6-hydroxy-9-oxo-1H,9H-benzopyrano[2,3-d]-v-triazole (0.865 g) in dry DMF (40 ml) and the mixture stirred for 15 minutes at room temperature. Triphenylmethylchloride (1.23 g) was added and stirring was continued for 2 days after which time the solvent was removed *in vacuo* and water added. After acidification to pH 4, the product was extracted into ethyl acetate and the dried ($MgSO_4$) extracts were evaporated to an oil. Chromatography on silica eluting with chloroform gave 0.755 g (40%) of title compound of mp 259—260°C (dec), $v_{max}$ (mull) 3400—2600, 1665, 1615, 1550 $cm^{-1}$; δ (DMSO-$d_6$) 7.07 (17H, m), 8.02 (1H, d, J 9 Hz), 11.06 (1H, broad exchangeable, singlet). Found: C, 75.36; H, 4.64; N, 9.28; $C_{28}H_{19}N_3O_3$ requires: C, 75.49; H, 4.30; N, 9.43%.

Increased yields (up to 62%) can be obtained by further addition of triphenylmethylchloride (0.5 equivalent) after 24 hours.

(g) 6-{3-[4-(4-Chlorobenzyl)-1-piperazinyl]-propyloxy}-9-oxo-2-triphenylmethyl-9H-benzopyrano[2,3-d]-v-triazole

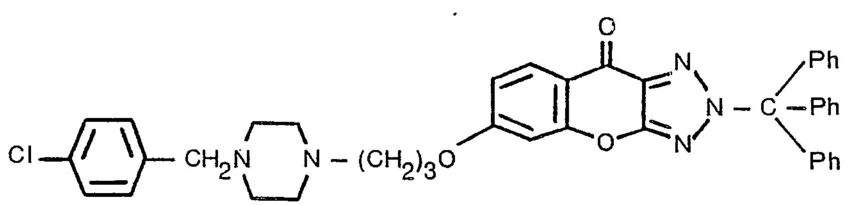

To a stirred solution of 6-hydroxy-9-oxo-2-triphenylmethyl-9H-benzopyrano[2,3-d]-v-triazole (11.0 g, 0.025 mole), triphenylphosphine (11.80 g) and 1-(4-chlorobenzyl)-4-(3-hydroxypropyl)piperazine (6.68 g, 0.025 mole) in dry THF (250 ml) was added diethyl azodicarboxylate (7.84 g, 0.045 mole) dropwise. After 1 hour at ambient temperature the solvent was evaporated *in vacuo* and the residue chromatographed on silica (dichloromethane → chloroform) to give 6.40 g (37%) of title compound of mp (EtOH-$CHCl_3$) 182°C: $v_{max}$ (mull), 1550, 1620, 1670 $cm^{-1}$; δ (DMSO-$d_6$) 2.03 (2H, m), 2.48 (10H, m), 3.47 (2H, s), 4.13 (2H, t, J 6 Hz), 6.90 (1H, broad s), 7.01 (1H, dd), 7.26 (19H, m), 8.28 (1H, d, J 9 Hz). Found: C, 71.87; H, 5.53; N, 9.85; $C_{42}H_{38}ClN_5O_3{\cdot}0.5H_2O$ requires C, 71.52; H, 5.57; N, 9.93%.

(h) 6-{3-[4-(4-Chlorobenzyl)-1-piperazinyl]-propyloxy}-9-oxo-1H,9H-benzopyrano[2,3-d]-v-triazole

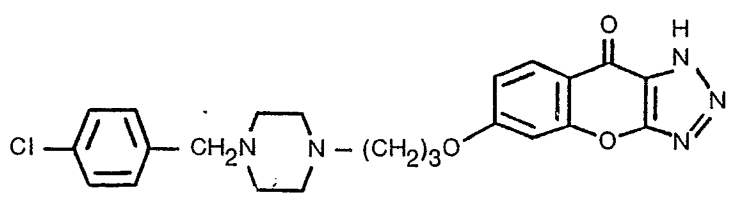

Concentrated hydrochloric acid (5 ml) was added to a stirred solution of 6-{3-[4-(4-chlorobenzyl)-1-piperazinyl]-propyloxy}-9-oxo-2-triphenylmethyl-9H-benzopyrano[2,3-d]-v-triazole (6.20 g) in glacial acetic

acid (200 ml) and the solution was stirred for 3 hours at 70°C. The solution was evaporated *in vacuo* and the residue suspended in water and neutralised to pH 6.5 with dilute sodium hydroxide. The white solid was filtered off and boiled with ethanol to remove trityl alcohol. Filtration gave 3.81 g (94%) of material of mp (EtOH) 210°C (dec).

Example 3

(a) 8-Hydroxy-9-oxo-1H,9H-benzopyrano[2,3-d]-v-triazole

A 50% dispersion of sodium hydride in mineral oil (3.84 g, 0.08 mole) was added to a solution of ethanethiol (5.10 g, 6.02 ml, 0.08 mole) in dry N,N-dimethylformamide (100 ml) under a nitrogen atmosphere and the mixture was stirred at ambient temperature for 15 minutes to complete the formation of the sodium salt. Finely powdered 8-methoxy-9-oxo-1H,9H-benzopyrano[2,3-d]-v-triazole (2.36 g, 0.011 mole) was then added and the orange solution was heated with stirring to 115°C (oil bath temperature) for 90 minutes. After cooling the mixture was poured into ice-water (400 ml) and the solution washed with ether. The filtered aqueous layer was acidified to pH 2 with hydrochloric acid to afford the title compound as an orange-pink precipitate. Recrystallization from methanol gave 1.18 g (53%) of pale orange solid of mp 256—258°C dec, $v_{max}$ (mull) 3160, 1638, 1618 $cm^{-1}$, δ (DMSO-$d_6$) 6.86 (1H, d, J 8 Hz); 7.14 (1H, d, J 8 Hz); 7.73 (1H, t, J 8 Hz); 12.30 (1H, exchangeable singlet).

(Found: C, 53.12; H, 2.60; N, 20.90; $C_9H_5N_3O_3$ requires: C, 53.21; H, 2.48; N, 20.69%)

(b) 8-Hydroxy-9-oxo-2-triphenylmethyl-9H-benzopyrano[2,3-d]-v-triazole

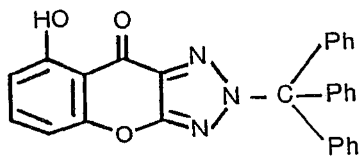

Anhydrous potassium carbonate (1.21 g, 8.8 mmole) followed by triphenylmethyl chloride (1.41 g, 4.9 mmole) was added to a solution of 8-hydroxy-9-oxo-1H,9H-benzopyrano[2,3-d]-v-triazole (0.96 g, 4.4 mmole) in dry N,N-dimethylformamide (40 ml) and the mixture was stirred at room temperature for 24 hours. The solvent was then evaporated *in vacuo* and the yellow residue diluted with water. After bringing the pH to 3 with dilute hydrochloric acid the yellow precipitate was filtered off and dried *in vacuo*. Chromatography on silica eluting with chloroform and then 20% ethanol in chloroform gave 1.51 g (77%) of material of mp 166—167°C, $v_{max}$ (mull) 1650, 1625, 1543 $cm^{-1}$, δ ($CDCl_3$) 6.80 (1H, d, J 8 Hz), 7.00 (1H, d, J 8 Hz), 7.23 (15H, m), 7.55 (1H, t, J 8 Hz), 12.36 (1H, exchangeable s).

(Found: C, 75.26; H, 4.51; N, 9.24; $C_{28}H_{19}N_3O_3$ requires: C, 75.49; H, 4.30; N, 9.43%).

(c) 8-{3-[4-(4-Chlorobenzyl)-1-piperazinyl]propyloxy}-9-oxo-2-triphenylmethyl-9H-benzopyrano[2,3-d]-v-triazole

The title compound can be prepared from the product of step (b) in similar manner to the process described in Example 1 (g).

(d) 8-{3-[4-(4-Chlorobenzyl)-1-piperazinyl]propyloxy}-9-oxo-1H,9H-benzopyrano[2,3-d]-v-triazole

The title compound can be prepared from the product of step (c) in similar manner to the process described in Example 1 (h).

Example 4

a) 2-Allyl-3-methoxyphenol and 2-allyl-5-methoxyphenol

A solution of allyl 3-methoxyphenyl ether (230 g, 1.40 mole; W N White and C D Slater, *J Org Chem, 26*, 3631—8 (1961)) in N,N-dimethylaniline (150 ml) was refluxed in an atmosphere of nitrogen for 4 hours. The orange solution was cooled, diluted with petroleum ether [bp 60—80°C] and washed with 5M sulphuric acid. The organic phase was extracted with 2.5M sodium hydroxide, and the aqueous extracts re-acidified with 5M hydrochloric acid. The isomeric mixture of phenols was extracted with ethyl acetate, dried ($MgSO_4$) and evaporated *in vacuo* to yield 144 g (63%) of brown oil the NMR of which showed 60% of 2-allyl-5-methoxy phenol, 40% of the other isomer. The two products could be separated by careful chromatography using silica gel, eluting with $CH_2Cl_2$/petroleum ether (bp 60—80°) [1:1] to yield the faster running 2-allyl-3-methoxyphenol as a colourless oil,

$v_{max}$ (film):

1598, 1610, 1637 (shoulder) 3475 $cm^{-1}$.

δ $CDCl_3$:

3.40 (2H, m);
3.80 (3H, s);
5.10 (3H, m);
5.95 (1H, m);

6.47 (2H, d, *J* 9 Hz);
7.30 (1H, t, *J* 9 Hz),

followed by the slower running 2-allyl-5-methoxyphenol as a pale brown oil,

$v_{max}$ (film):
1598, 1620, 1635 (weak) 3410 $cm^{-1}$.

δ $CDCl_3$:
3.31 (2H, m);
3.78 (3H, s);
5.23 (3H, m);
6.03 (1H, m);
6.40 (1H, bs);
6.47 (1H, dd, J 2 Hz and 9 Hz);
6.98 (1H, d, J 9 Hz).

b) Ethyl 5-(2-allyl-3-methoxyphenoxy)-1-(4-methoxybenzyl)-v-triazole-4-carboxylate

A 50% dispersion of sodium hydride in mineral oil (3.90 g, 0.08M) was added to a stirred solution of 2-allyl-3-methoxyphenol (13.32 g, 0.08 Mole) in dry DMF (300 ml) and to the resulting sodium salt was added ethyl-5-chloro-1-(4-methoxybenzyl)-v-triazole-4-carboxylate (24.0 g, 0.08 Mole), (D. R. Buckle and C. J. M. Rockell, J. Chem. Soc. Perkin Trans. I, 1982, 627). The reaction mixture was heated with stirring at 80°C for 18 hours and then cooled. Removal of the DMF *in vacuo* gave a red oil which was taken up in ethyl acetate, washed with water, and dried ($MgSO_4$). Evaporation gave an oil which crystallised on trituration. Recrystallisation from ethanol/petroleum ether [bp 60—80°C] gave 21.58 g (64%) of product, mp 109—10°C.

$v_{max}$ (mull):
1570, 1595, 1605, 1715 $cm^{-1}$.

δ $CDCl_3$:
1.02 (3H, t, *J* 6 Hz);
3.48 (2H, m);
3.73 (3H, s);
3.83 (3H, s);
4.09 (2H, q, *J* 6 Hz);
4.97 (2H, m);
5.28 (2H, s);
5.87 (1H, d, *J* 9 Hz);
5.90 (1H, m);
6.61 (1H, d, *J* 9 Hz);
6.92 (1H, t, *J* 9 Hz);
6.93 (4H, AB quartet Δv = 39 Hz, J 9 Hz).

Found: C, 64.87; H, 5.57; N, 9.86. $C_{23}H_{25}N_3O_5$ requires: C, 65.25; H, 5.95; N, 9.92%. $M^+$ = 423.1822 ($C_{23}H_{25}N_3O_5$).

c) Ethyl 5-(3-methoxy-2-n-propylphenoxy)-1H-v-triazole-4-carboxylate

Hydrogenolysis of a solution of the above compound (1.0 g) in ethanol (300 ml) over 10% palladium on charcoal at 100°C and 1 000 psi resulted in reduction of the allyl double bond and clean removal of the N-(4-methoxybenzyl)group within 10 hours. On cooling and removal of the catalyst by filtration, evaporation *in vacuo* gave an oil which was purified by column chromatography to yield 0.53 g (63%) of product, a white solid, mp 97—9°C.

$v_{max}$ (mull):
1585, 1603, 1690, 3180 $cm^{-1}$.

δ ($CDCl_3$):
0.87 (3H, t, *J* 7 Hz);
1.26 (3H, t, *J* 7 Hz);
1.50 (2H, m);
2.63 (2H, t, *J* 7 Hz);
3.82 (3H, s);
4.33 (2H, q, *J* 7 Hz);
6.63 (2H, m);
7.06 (1H, t, *J* 8 Hz).

Found: C, 58.62; H, 6.42; N, 13.54; $C_{15}H_{19}N_3O_4$ requires C, 59.00; H, 6.27; N, 13.76%. $M^+$ =305.1388 ($C_{15}H_{19}N_3O_4$).

d) 5-(3-Methoxy-2-n-propylphenoxy)-1H-v-triazole-4-carboxylic acid

Hydrolysis of ethyl 5-(3-methoxy-2-n-propylphenoxy)-1H-v-triazole-4-carboxylate (1.06 g) with 1.0M sodium hydroxide solution (15 ml) at 70°C over 2 hours, afforded the acid which was isolated by acidification of the cooled solution. Recrystallisation from aqueous ethanol gave 0.84 g (88%) of product, mp 146—7°C (dec).

$\nu_{max}$ (mull):
1595, 1608, 1695, 3200 $cm^{-1}$.
δ DMSO-$d_6$:
0.80 (3H, t, *J* 6 Hz);
1.43 (2H, m);
2.52 (2H, m);
3.79 (1H, s);
6.48 (1H, d, *J* 8 Hz);
6.73 (1H, d, *J* 8 Hz);
7.08 (1H, t, *J* 8 Hz).
Found: C, 56.63; H, 5.47; N, 14.81. $C_{13}H_{15}N_3O_4$ requires: C, 56.31; H, 5.45; N, 15.16%. $M^+$ = 277.1052 ($C_{13}H_{15}N_3O_4$).

e) 6-Methoxy-9-oxo-5-n-propyl-1H,9H-benzopyrano[2,3-d]-v-triazole

To a solution of phosphoric oxide (8.0 g) in 98% methanesulphonic acid (20 ml), at 80°C was added 5-(3-methoxy-2-n-propylphenoxy)-1H-v-triazole-4-carboxylic acid (0.94 g, 3.4 mmole) with stirring and the pale red solution maintained at 80°C for 3 hours, after which time HPLC showed a trace of starting material remaining. The reaction was cooled, diluted with water (100 ml), and the resultant brick red precipitate filtered off, washed with water and dried *in vacuo*. Recrystallisation from aqueous ethanol afforded 0.40 g (46%) of product as a cream solid, mp 220—2°C. (dec).
$\nu_{max}$ (mull):
1610, 1660 $cm^{-1}$.
δ DMSO-$d_6$:
0.94 (3H, t, *J* 7 Hz);
1.60 (2H, m);
2.81 (2H, t, *J* 7 Hz);
3.98 (3H, s);
7.21 (1H, d, *J* 9 Hz);
8.08 (1H, d, *J* 9 Hz).
Found: C, 60.10; H, 5.03; N, 16.10. $C_{13}H_{13}N_3O_3$ requires: C, 60.22; H, 5.05; N, 16.2%. $M^+$ = 259.0956 ($C_{13}H_{13}N_3O_3$).

f) 6-Hydroxy-9-oxo-5-n-propyl-1H,9H-benzopyrano[2,3-d]-v-triazole

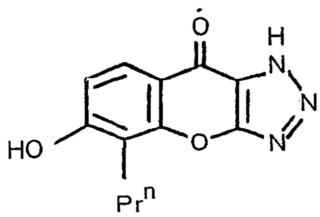

A 50% dispersion of sodium hydride in mineral oil (12.20 g, 0.25M) was added to a stirred solution of ethanethiol (15.76 g, 0.25M) in dry DMF (250 ml) and to the resulting sodium salt was added 6-methoxy-9-oxo-5-n-propyl-1H,9H-benzopyrano-[2,3-d]-v-triazole (9.40 g, 0.036M). The mixture was heated with stirring to 120°C for 0.75 hours when HPLC showed no starting material. The reaction was cooled, evaporated *in vacuo* to dryness and the residue poured into water. The product was isolated by acidification of the solution. Recrystallisation from ethanol gave 5.84 g (66%) of pale yellow crystals, mp 283—4°C (dec).
$\nu_{max}$ (mull):
1550, 1560, 1605, 1640, 3130 $cm^{-1}$.
δ DMSO-$d_6$:
1.00 (3H, t, *J* 7 Hz);
1.64 (2H, m);
2.84 (2H, t, *J* 7 Hz);
7.05 (1H, d, *J* 9 Hz);
7.98 (1H, d, *J* 9 Hz);
11.00 (1H, bs, exchanged $D_2O$);
16.45 (1H, bs, exchanged $D_2O$).
Found: C, 58.55; H, 4.74; N, 17.03. ($C_{12}H_{11}N_3O_3$ requires: C, 58.77; H, 4.52; N, 17.14%) $M^+$ = 245.0799 ($C_{12}H_{11}N_3O_3$).

(g) 6-Hydroxy-9-oxo-5-n-propyl-2-triphenylmethyl-9H-benzopyrano[2,3-d]-v-triazole

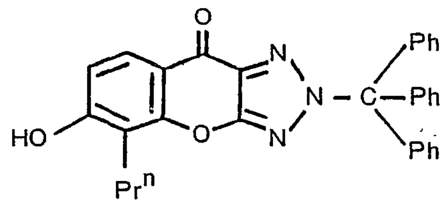

Triphenylmethylchloride (2.35 g, 8.2 mmole) was added to a stirred mixture of anhydrous potassium carbonate (1.67 g, 12 mmole) and 6-hydroxy-9-oxo-n-propyl-1H,9H-benzopyrano[2,3-d]-v-triazole (2.0 g, 8.2 mmole) in dry DMF (50 ml) and the mixture stirred at room temperature for 24 hours. The solvent was evaporated to dryness *in vacuo* and the residue suspended in water. After acidification the product was extracted with chloroform. The evaporated, dried extracts were chromatographed on silica eluting with dichloromethane to give 1.36 g (34%) of title compound of mp(ethanol) 221—222°C (dec), $\nu_{max}$ (mull) 1555, 1600, 1605, 1660, 3330 $cm^{-1}$; δ (DMSO-$d_6$) 0.93 (3H, t, J 7 Hz), 1.56 (2H, m), 2.78 (2H, t, J 7 Hz), 7.01 (1H, d, J 9 Hz), 7.28 (15H, m), 7.94 (1H, d, J 9 Hz), 10.93 (1H, broad s, exchangeable). $M^+$ 487.1889 ($C_{31}H_{25}N_3O_3$).

(h) 6-{3-[4-(4-Chlorobenzyl)-1-piperazinyl]propyloxy}-9-oxo-5-n-propyl-2-triphenylmethyl-9H-benzopyrano[2,3-d]-v-triazole

The title compound can be prepared from the product of step (g) in a similar manner to the process described in Example 1 (g).

(i) 6-{3-[4-(4-Chlorobenzyl)-1-piperazinyl]propyloxy}-9-oxo-5-n-propyl-1H,9H-benzopyrano[2,3-d]-v-triazole

The title compound can be prepared from the product of step (h) in a similar manner to the process described in Example 1 (h).

Example 5

a) Ethyl 5-(2-allyl-5-methoxyphenoxy)-1-benzyl-v-triazole-4-carboxylate

To a solution of 2-allyl-5-methoxyphenol (15.41 g, 0.094 mmole) in dry DMF (150 ml) was added a 50% dispersion of sodium hydride in mineral oil (4.51 g, 0.094 mole) and the mixture stirred at ambient temperature for 15 minutes to complete formation of the sodium salt. Ethyl 1-benzyl-5-chloro-v-triazole-4-carboxylate (27.8 g, 0.094 mole) was added and the reaction mixture stirred at 70—80°C for 16 hours. After cooling, the solvent was removed *in vacuo* and the product was partitioned between water and ethyl acetate. The organic phase was washed with dilute sodium hydroxide, water and brine and dried ($MgSO_4$). Evaporation and chromatography of the residual oil on silica with chloroform elution gave 32.54 g (88%) of the pure product. Recrystallisation from toluene-petroleum ether [bp 40—60°] gave a white solid of mp 55—56°C.

$\nu_{max}$ (mull)
1738, 1620, 1555, 1505 $cm^{-1}$.
δ ($CDCl_3$):
1.08 (3H, t, *J* 7.3 Hz);
3.41 (2H, d, *J* 5.4 Hz);
3.52 (3H, s);
4.18 (2H, q, *J* 7.3 Hz);
4.98 (1H, d);
5.15 (1H, d);
5.40 (1H, s);
5.78 (1H, d, *J* 2.7 Hz);
6.00 (1H, complex m);
6.60 (1H, d.d, J 8.4 Hz, 2.7 Hz);
7.14 (1H, d, *J* 8.4 Hz); 7.25 (5H, s).
Found: C, 67.12; H, 6.00; N, 10.73. $C_{22}H_{23}N_3O_4$ requires: C, 67.16; H, 5.89; N, 10.68%.

b) Ethyl 5-(5-methoxy-2-n-propylphenoxy)-1H-v-triazole-4-carboxylate

Hydrogenolysis of a solution of ethyl 5-(2-allyl-5-methoxyphenoxy)-1-benzyl-v-triazole-4-carboxylate (30 g) in ethanol (300 ml) over 10% palladinized charcoal (1 g) at 100°C and 1 000 psi gave the title compound after 5 hours. Filtration and evaporation of the solution gave an oil which was purified on silica chromatography eluting with chloroform to give 22.73 g (98%) of product as a pale yellow oil.

$\nu_{max}$ (film):
3200, 1720, 1620 $cm^{-1}$.
δ ($CDCl_3$):
0.92 (3H, t, J 7 Hz);
1.32 (3H, t, J 7.3 Hz);
1.60 (2H, sextet, J 8 Hz);
2.60 (2H, t, J 8 Hz);

3.77 (3H, s);
4.41 (2H, q, J 7.3 Hz);
6.65 (1H, d, J 2.7 Hz);
6.70 (1H, d.d, J 8.4 Hz, 2.7 Hz);
7.15 (1H, d, J 8.4 Hz);
9.5 (1H, broad exchangeable).

c) 5-(5-Methoxy-2-n-propylphenoxy)-1H-v-triazole-4-carboxylic acid

A solution of ethyl 5-(5-methoxy-2-n-propylphenoxy)-1H-v-triazole-4-carboxylate (22 g, 0.072 mole) in 1M aqueous sodium hydroxide (330 ml) was stirred at 70°C for 2 hours, cooled and washed once with ethyl acetate. The aqueous phase was brought to pH 1 at 0°C and the precipitated oil extracted into ethyl acetate. The dried extracts were evaporated to give 19.79 g (98%) of crude material. Trituration with toluene-ether followed by petroleum ether [bp 40—60°] gave 12.56 g of compound of mp 112—3°C (dec). Chromatography of the concentrated mother liquors on silica eluting with chloroform then 10% methanol in chloroform gave a further 2.76 g of material. Recrystallisation from ethyl acetate/petroleum ether gave mp 113—4°C.

$v_{max}$ (mull):
3170, 2650 (broad), 1720, 1620, 1530, 1505 $cm^{-1}$.
δ (DMSO-$d_6$):
0.88 (3H, t, J 6 Hz);
1.55 (2H, sextet, J 6 Hz);
2.56 (2H, t, J 6 Hz);
3.70 (3H, s);
6.57 (1H, d, J 2 Hz);
6.73 (1H, d.d, J 2 Hz, 8 Hz);
7.22 (1H, d, J 8 Hz);
14.3 (2H, broad exchangeable signal).
Found: C, 55.97; H, 5.52; N, 14.92. $C_{13}H_{15}N_3O_4$ requires: C, 56.31; H, 5.45; N, 15.16%.

d) 8-Methoxy-9-oxo-5-n-propyl-1H,9H-benzopyrano[2,3-d]-v-triazole

To a solution of phosphoric oxide (8.0 g) in 98% methanesulphonic acid (20 ml) was added finely powdered 5-(5-methoxy-2-n-propylphenoxy)-1H-v-triazole-4-carboxylic acid (0.94 g, 3.4 mmole) and the mixture was stirred at 80°C for 1.5 hours when no acid remained. The red solution was cooled and diluted to 200 ml with water. The bulk of the acidic solution was neutralized and the precipitated solid filtered off and recrystallised from ethanol to give 0.62 g (70%) of title compound of mp 229—231°C (dec).

$v_{max}$ (mull):
3050 (broad), 1670, 1597, 1530 $cm^{-1}$.
δ (DMSO-$d_6$):
0.96 (3H, t, J 7 Hz);
1.66 (2H, sextet, J 7 Hz);
2.77 (2H, t, J 7.5 Hz);
3.92 (3H, s);
6.99 (1H, d, J 9 Hz);
7.62 (1H, d, J 9 Hz).
Found: C, 60.18; H, 5.02; N, 16.13. $C_{13}H_{13}N_3O_3$ requires: C, 60.22; H, 5.05; N, 16.21%.

e) 8-Hydroxy-9-oxo-5-n-propyl-1H,9H-benzopyrano[2,3-d]-v-triazole

To a solution of ethane thiol (10.2 g, 12.05 ml, 0.16 mole) in dry DMF (200 ml) was added a 50% dispersion of sodium hydride in mineral oil (7.65 g, 0.16 mole) and the mixture was stirred for 15 minutes at ambient temperature to complete formation of the sodium salt. Finely powdered 8-methoxy-9-oxo-5-n-propyl-1H,9H-benzopyrano[2,3-d]-v-triazole (5.66 g, 0.022 mole) was added in one portion and the total was stirred at 120°C (oil bath temperature) for 1 hour. The DMF was removed *in vacuo* and water (250 ml) was added to the residue. After washing the resulting solution with ether the solution was cooled to 0°C and acidified. The precipitated oil crystallized on trituration to give a yellow solid of tlc purity which was filtered off, washed with water and dried to give 5.09 g (95%) of the title compound of mp 208—9°C (dec) (from ethyl acetate).

$v_{max}$ (mull):
3130 (broad), 1650, 1638, 1618, 1560, 1525 $cm^{-1}$.
δ (DMSO-$d_6$):
0.95 (3H, t, J 7.2 Hz);
1.68 (2H, sextet, J 7 Hz);
2.78 (3H, t, J 7 Hz);

6.82 (1 H, d, J 8 Hz);
7.62 (1H, d, J 8 Hz);
12.24 (1H, sharp exchangeable s);
15.2 (1H, broad exchangeable).
(Found: C, 59.08; H, 4.41; N, 16.83. $C_{12}H_{11}N_3O_3$ requires: C, 58.77; H, 4.52; N, 17.14%)

f) 8-Hydroxy-9-oxo-5-n-propyl-2-(triphenylmethyl)-9H-benzopyrano[2,3-d]-v-triazole

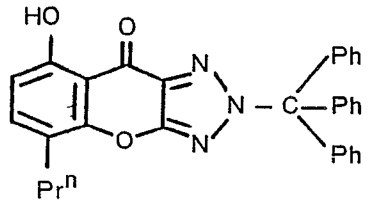

Anhydrous potassium carbonate (1.04 g) and triphenylmethyl chloride (1.39 g, 5 mmole) were added to a solution of 8-hydroxy-9-oxo-5-n-propyl-1H,9H-benzopyrano[2,3-d]-v-triazole (1.23 g, 5 mmole) in dry DMF (50 ml) and the mixture was stirred at 60°C for 18 hours. After cooling the solvent was removed *in vacuo* and water added. After bringing the pH to 6 with hydrochloric acid the yellow solid was filtered off and dried to give 2.26 g of crude title compound. Chromatography on silica eluting with dichloromethane gave 1.60 g (69%) of title compound as a yellow solid, mp (ethanol-chloroform) 194—5°C.
$\nu_{max}$ (mull):
1662, 1625, 1550 $cm^{-1}$.
δ ($CDCl_3$):
0.98 (3H, t, J 7.2 Hz);
1.66 (2H, sextet, J 7.2 Hz);
2.77 (2H, t, J 7.2 Hz);
6.82 (1H, d, J 8 Hz);
7.3 (15H, m);
7.48 (1H, d, J 8 Hz);
12.33 (1H, sharp exchangeable s).
(Found: C, 74.85; H, 5.46; N, 8.27. $C_{31}H_{25}N_3O_3{\cdot}0.5H_2O$ requires: C, 74.98; H, 5.78; N, 8.46%)
From the aqueous phase was obtained 0.36 g of starting phenol by acidification to pH 1.

g) 8-{3-[4-(4-Chlorobenzyl)-1-piperazinyl]propyloxy}-9-oxo-5-n-propyl-2-triphenylmethyl-9H-benzopyrano[2,3-d]-v-triazole

The title compound can be prepared from the product of step (f) in a similar manner to the process described in Example 1 (g).

h) 8-{3-[4-(4-Chlorobenzyl)-1-piperazinyl]propyloxy}-9-oxo-5-n-propyl-1H,9H-benzopyrano[2,3-d]-v-triazole

The title compound can be prepared from the product of step (g) in a similar manner to the process described in Example 1 (h).

**Claims**

1. A compound of formula (I):

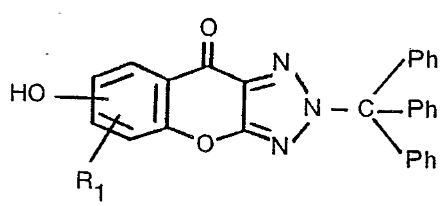

(I)

wherein $R_1$ is hydrogen or $C_{1-6}$ alkyl, and each phenyl group is optionally substituted by one or two groups selected from $C_{1-3}$ alkyl, $C_{1-3}$ alkoxy and halogen.

2. A compound according to claim 1, wherein any substituent on a phenyl group is in the meta- or para-position.

3. A compound according to claim 1, wherein each phenyl group is unsubstituted.

4. A compound according to any one of claims 1 to 3, wherein $R_1$ is in the 5-position.

5. A compound according to any one of claims 1 to 4, wherein the OH group is in the 6-position.

6. A compound selected from 6-hydroxy-5-methyl-9-oxo-2-triphenylmethyl-9H-benzopyrano[2,3-d]-v-triazole; and 8-hydroxy-9-oxo-2-triphenylmethyl-9H-benzopyrano[2,3-d]-v-triazole.

7. A process for preparing a compound of formula (I) according to claim 1, in which a compound of formula (II):

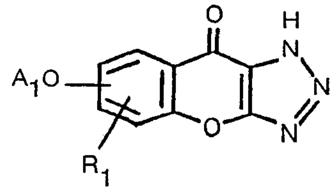

(II)

wherein $R_1$ is as defined in claim 1, $A_1$ is hydrogen or an oxygen protecting group, is reacted with a triphenylmethyl halide wherein each phenyl group is optionally substituted as defined in claim 1; and thereafter if necessary removing any oxygen protecting group $A_1$ in conventional manner.

8. A process for preparing a compound of formula (V)

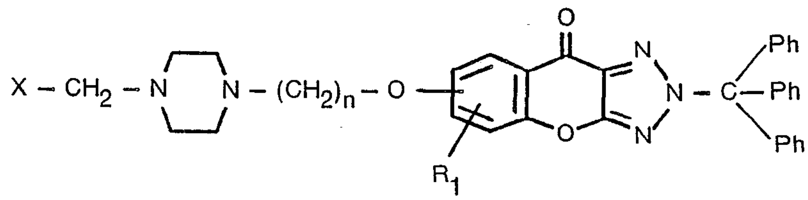

(V)

wherein X is phenyl optionally substituted by one halogen, $C_{1-4}$ alkyl or $C_{1-4}$ alkoxy; or pyridyl; $R_1$ is hydrogen or $C_{1-6}$ alkyl; n is 1 to 6; and each phenyl group is optionally substituted as defined in formula (I); which process comprises coupling a compound of formula (I) as defined in claim 1 with a compound of formula (IV)

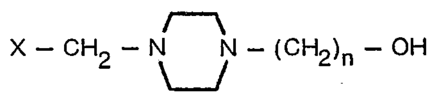

(IV)

wherein X is phenyl, optionally substituted by one halogen, $C_{1-4}$ alkyl or $C_{1-4}$ alkoxy; or pyridyl; and n is 1 to 6.

**Patentansprüche**

1. Eine Verbindung der Formel (I):

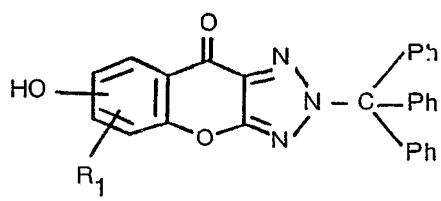

(I)

in der R Wasserstoff doer $C_{1-6}$-Alkyl ist und jede Phenylgruppe gegebenenfalls durch eine Gruppe oder zwei Gruppen substituiert ist, die aus $C_{1-3}$-Alkyl, $C_{1-3}$-Alkoxy und Halogen ausgewählt ist(sind).

2. Eine Verbindung nach Anspruch 1, wobei irgendein Substituent an einer Phenylgruppe in der m-oder p-Stellung steht.

3. Eine Verbindung nach Anspruch 1, wobei jede Phenylgruppe unsubstituiert ist.

4. Eine Verbindung nach irgendeinem der Ansprüche 1 bis 3, wobei $R_1$ in der 5-Stellung steht.

5. Eine Verbindung nach irgendeinem der Ansprüche 1 bis 4, wobei die OH-Gruppe in der 6-Stellung steht.

6. Eine Verbindung ausgewählt aus 6-Hydroxy-5-methyl-9-oxo-2-triphenylmethyl-9H-benzopyrano[2,3-d]-v-triazol und 8-Hydroxy-9-oxo-2-triphenylmethyl-9H-benzopyrano[2,3-d]-v-triazol.

7. Ein Verfahren zur Herstellung einer Verbindung der Formel (I) nach Anspruch 1, wobei eine Verbindung der Formel (II)

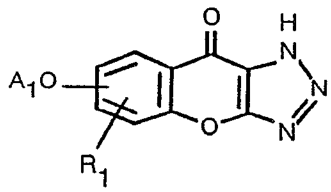

(II)

in der $R_1$ wie in Anspruch 1 definiert ist und $A_1$ Wasserstoff oder eine Sauerstoffschutzgruppe ist, mit einem Triphenylmethylhalogenid, bei dem jede Phenylgruppe gegebenenfalls wie in Anspruch 1 definiert substituiert ist, umsetzt und erforderlichenfalls anschließend in üblicher Weise irgendeine Sauerstoffschutzgruppe $A_1$ entfernt.

8. Ein Verfahren zur Herstellung einer Verbindung der Formel (V)

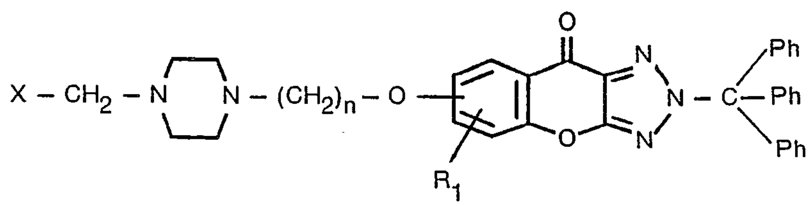

(V)

in der X Phenyl, das gegebenenfalls durch ein Halogen, $C_{1-4}$-Alkyl oder $C_{1-4}$-Alkoxy substituiert ist, oder Pyridyl ist, $R_1$ Wasserstoff oder $C_{1-6}$-Alkyl ist, n den Wert 1 bis 6 hat und jede Phenylgruppe gegebenenfalls wie bei Formel (I) definiert substituiert ist, wobei das Verfahren das Kuppeln einer Verbindung der Formel (I) wie in Anspruch 1 definiert mit einer Verbindung der Formel (IV):

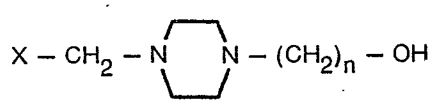

(IV)

in der X Phenyl, das gegebenenfalls durch ein Halogen, $C_{1-4}$-Alkyl oder $C_{1-4}$-Alkoxy substituiert ist, oder Pyridyl ist und n den Wert 1 bis 6 hat, umfaßt.

## Revendications

1. Composé représenté par la formule (I):

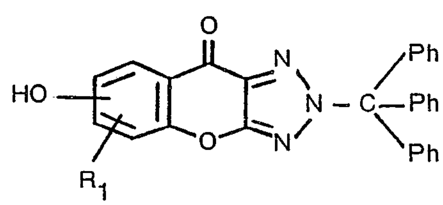

(I)

dans laquelle $R_1$ est un atome d'hydrogène ou un groupe alcoyle en $C_{1-6}$ et chaque groupe phényle est éventuellement substitué par un ou deux groupes choisis à partir de groupes alcoyles en $C_{1-3}$, alcoxy en $C_{1-3}$ et des atomes d'halogène.

2. Composé suivant la revendication 1, caractérisé en ce qu'un quelconque substituant du groupe phényle est en position méta- ou para.

3. Composé suivant la revendication 1, caractérisé en ce que chaque groupe phényle est non-substitué.

4. Composé suivant l'une quelconque des revendications 1 à 3, caractérisé en ce que $R_1$ est en position 5.

5. Composé suivant l'une quelconque des revendications 1 à 4, caractérisé en ce que le groupe —OH est en position 6.

6. Composé choisi parmi le 6-hydroxy-5-méthyl-9-oxo-2-triphénylméthyl-9H-benzopyrano[2,3-d]-v-triazole; et le 8-hydroxy-9-oxo-2-triphénylméthyl-9H-benzopyrano[2,3-d]-v-triazole.

7. Procédé de préparation d'un composé de formule (I) suivant la revendication 1, caractérisé en ce qu'on fait réagir un composé de formule (II)

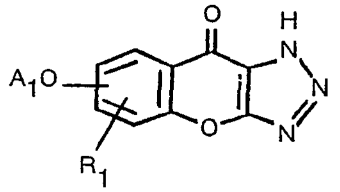

(II)

dans laquelle $R_1$ est tel que défini dans la revendication 1, $A_1$ est un atome d'hydrogène ou un groupe protégeant l'atome d'oxygène, avec un halogénure de triphénylméthyle dans lequel chaque groupe phényle est éventuellement substitué de la façon qui est définie dans la revendication 1; et ensuite, si cela est nécessaire, on élimine un quelconque groupe $A_1$ protégeant l'atome d'oxygène selon une méthode classique.

8. Procédé de préparation d'un composé de formule (V)

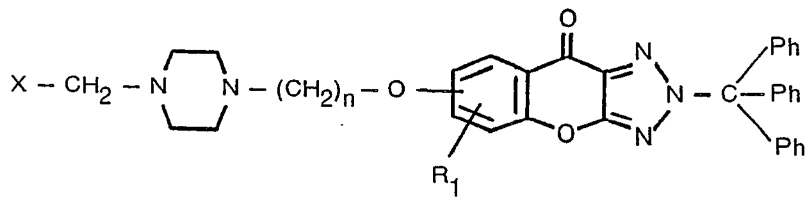

(V)

dans laquelle X est un groupe phényle éventuellement substitué par un atome d'halogène, un groupe alcoyle en $C_{1-4}$ ou alcoxy en $C_{1-4}$; ou pyridyle; $R_1$ est un atome d'hydrogène ou un groupe alcoyle en $C_{1-6}$; n est 1 à 6; et chaque groupe phényle est éventuellement substitué de la façon qui est définie dans la formule (I), caractérisé en ce que l'on couple un composé de formule (I) tel que défini dans la revendication 1, avec un composé de formule (IV)

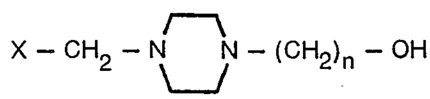

(IV)

dans laquelle X est un groupe phényle éventuellement substitué par un atome d'halogène, un groupe alcoyle en $C_{1-4}$ ou alcoxy en $C_{1-4}$; ou un groupe pyridyle; et n est 1 à 6.